# EUROPEAN PATENT APPLICATION

(11) **EP 2 591 728 A1**
(43) Date of publication of application: **15.05.2013**
(21) Application number: 12191914.6
(22) Date of filing: 09.11.2012
(51) Int. Cl.: A61B 6/00, A61B 6/06

(54) **Computed tomography apparatus and control method for the same**

(30) Priority: 14.11.2011 KR 20110118051
(71) Applicant: Samsung Electronics Co., Ltd, Gyeonggi-do 443-742 (KR)
(72) Inventor: Cho, Min Kook, Gyeonggi-do (KR); Kim, Ki Yeo, Gyeonggi-do (KR); Choi, Byung Sun, Gyeonggi-do (KR)
(74) Representative: D'Halleweyn, Nele Veerle Trees Gertrudis

(57) **Abstract**

A computed tomography apparatus and a method for controlling the same are provided. The apparatus is capable of switching between a high energy mode and a low energy mode at a high speed using a filter that rotates at a high speed. The computed tomography apparatus includes an X-ray generator which generates and irradiates X-rays toward a subject, an X-ray filter which includes at least one filter member, a driver which rotates the X-ray filter such that the filter members are selectively disposed in an irradiation path of X-rays generated by the X-ray generator, a detector which detects the X-rays that are transmitted to the subject, and a host apparatus which obtains X-ray images by using detected X-rays, separates the obtained X-ray images based on filter members to which the corresponding X-rays are transmitted, and reconstructs the images by using X-ray images of the identical filter member.

## Description

### BACKGROUND

### 1. Field

Apparatuses and methods consistent with exemplary embodiments relate to a computed tomography apparatus comprising an X-ray filter for filtering X-rays based on a corresponding energy range and a method for controlling the same.

### 2. Description of the Related Art

An X-ray imaging apparatus is an apparatus that irradiates an X-ray toward a subject, analyzes the X-ray that passes through the subject, and thereby determines information relating to an inner structure of the subject. Because the transmittance of an X-ray varies based on the tissue constituting the subject, an inner structure of the subject can be imaged by using an attenuation coefficient obtained by quantifying the same.

An X-ray imaging apparatus may be classified as one of a simple X-ray imaging apparatus which irradiates an X-ray in one direction and a computed tomography (CT) apparatus which irradiates X-rays in various directions and thereby reconstructs an image by using a computer. A CT apparatus is generally referred to as a computed tomography apparatus or a computerized tomography apparatus.

Recently, methods for obtaining X-ray images with superior contrast by irradiating X-rays at different energy levels, rather than irradiating X-rays at a single X-ray energy level, have been developed, and a significant amount of research associated therewith is currently underway.

However, when a plurality of X-ray generators and detectors are used in order to generate X-rays having different energy levels, signals may be detected in other X-ray detectors due to scattered lines that occur in the body and may disadvantageously act as noise, because a plurality of X-ray images can be simultaneously obtained.

Further, problems may occur in which a separate process for generalizing and adjusting properties of a plurality of systems is required, and therefore, it may be difficult to obtain uniform results at respective energy ranges, because a high voltage should be switched within a relatively short time when an energy mode is switched between a high energy mode and a low energy mode at a high speed in one X-ray generator.

### SUMMARY

One or more exemplary embodiments provide a computed tomography apparatus and a method for controlling the same, which apparatus is capable of switching between a high energy mode and a low energy mode at a high speed using a filter that rotates at a high speed, while an X-ray generator generates X-rays having a uniform energy.

In accordance with an aspect of an exemplary embodiment, there is provided a computed tomography apparatus including: an X-ray generator which generates X-rays and irradiates the generated X-rays toward a subject; an X-ray filter disposed between the X-ray generator and the subject, the X-ray filter including at least one filter member which filters an X-ray having an energy level which falls within a predetermined energy range; a driver which rotates the X-ray filter such that the at least one filter member is selectively disposed in an irradiation path of X-rays generated by the X-ray generator; a detector which detects the X-rays that are irradiated by the X-ray generator and filtered by the X-ray filter; and a host apparatus which obtains X-ray images by using X-rays detected by the detector, separates the obtained X-ray images based on a particular filter member from among the at least one filter member by which the corresponding X-rays are filtered, and reconstructs the separated images based on a respective X-ray energy range by using X-ray images corresponding to the particular filter member.

The host apparatus may separate the obtained X-ray images based on a disposition order of the at least one filter member and an acquisition order of the X-ray images.

A rotational speed of the X-ray filter may relate to a time required for obtaining at least one X-ray image by the host apparatus.

A time at which the at least one X-ray filter member is disposed in the X-ray irradiation path may be synchronized with the time required for obtaining the at least one X-ray image by the host apparatus.

The X-ray filter may have a circular shape and may include a rotation shaft which is rotated by the driver, and the at least one X-ray filter member may be disposed based on the rotation shaft.

The X-ray generator may receive a voltage having a predetermined level for generating an X-ray.

The X-ray filter may include an X-ray blocker which blocks X-rays.

In accordance with an aspect of another exemplary embodiment, there is provided a method for controlling a computed tomography apparatus including: generating X-rays and irradiating the generated X-rays toward a subject; filtering the irradiated X-rays by using an X-ray filter that includes at least one filter member which filters an X-ray having an energy level which falls within a predetermined energy range and which rotates at a predetermined rotational speed; detecting the filtered X-rays; obtaining X-ray images by using the detected X-rays; separating the obtained X-ray images based on a particular filter member from among the at least one filter member which filters the corresponding X-rays; and reconstructing the separated images based on a respective X-ray energy range by using X-ray images corresponding to the particular filter member.

The obtained X-ray images may be separated based on a disposition order of the at least one filter member and an acquisition order of the X-ray images.

The predetermined rotational speed of the X-ray filter may relate to a time required for obtaining at least one X-ray image.

A time at which the at least one X-ray filter member is disposed in an X-ray irradiation path may be synchronized with the time required for obtaining the at least one X-ray image.

The X-ray filter may have a circular shape and include a rotation shaft which is rotated by a driver, and the at least one X-ray filter member may be disposed based on the rotation shaft.

### BRIEF DESCRIPTION OF THE DRAWINGS

The above and/or other aspects will become apparent and more readily appreciated from the following description of the exemplary embodiments, taken in conjunction with the accompanying drawings of which:
FIG. 1 is a block diagram which illustrates a controlled configuration of a computed tomography apparatus according to an exemplary embodiment;
FIG. 2A is a graph which illustrates a level of power supplied from a power supply to an X-ray generator in a computed tomography apparatus of the related art, and FIG. 2B is a graph which illustrates a level of power supplied from a power supply to an X-ray generator in a computed tomography apparatus according to an exemplary embodiment;
FIG. 3A is a graph which illustrates an energy profile of an X-ray irradiated from an X-ray generator of a computed tomography apparatus of the related art to a subject, and
FIG. 3B is a graph which illustrates an energy profile of an X-ray passing through the X-ray filter 130 of the computed tomography apparatus according to an exemplary embodiment;
FIG. 4A is a perspective view which illustrates a computed tomography apparatus according to an exemplary embodiment, and FIG. 4B is a cross-sectional view which illustrates a computed tomography apparatus according to an exemplary embodiment;
FIG. 5 is a front view which illustrates a configuration of an X-ray filter of the computed tomography apparatus according to an exemplary embodiment;
FIG. 6 is a block diagram which illustrates a controlled configuration of a computed tomography apparatus according to an exemplary embodiment;
FIG. 7A and FIG. 7B are diagrams which illustrate images of X-rays that pass through respective filter members, separated at the respective filter members; and FIG. 8 is a sectional view of an X-ray filter and an accompanying chart which illustrates respective images of X-rays of FIGs. 7A and 7B with respect to the corresponding filter members and the corresponding acquisition angles of the X-ray images;
FIG. 9 is a block diagram which illustrates a controlled configuration of a computed tomography apparatus according to an exemplary embodiment;
FIG. 10 is a view which illustrates a relation of rotation speed of an X-ray filter to an image acquisition time; and
FIG. 11 is a flowchart which illustrates a method for controlling the computed tomography apparatus according to an exemplary embodiment.

### DETAILED DESCRIPTION

Hereinafter, a computed tomography apparatus according to exemplary embodiments will be described with reference to the accompanying drawings.

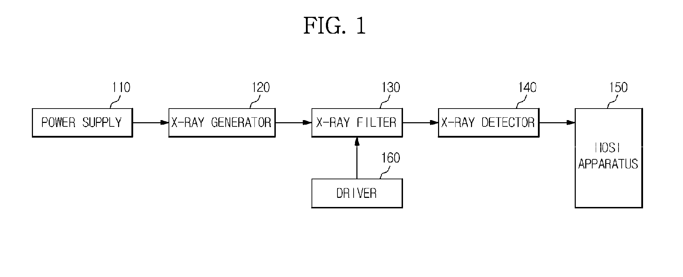

FIG. 1 is a block diagram which illustrates a controlled configuration of a computed tomography apparatus according to an exemplary embodiment.

Referring to FIG. 1, the computed tomography apparatus according to an exemplary embodiment includes an X-ray generator 120 which generates X-rays, a power supply 110 which supplies power to the X-ray generator, an X-ray filter 130 which filters X-rays having an energy level which falls within a predetermined energy range from among X-rays generated by the X-ray generator, a driver 160 which rotates the X-ray filter, a detector 140 which detects X-rays irradiated toward a subject via the X-ray filter, and a host apparatus 150 which obtains an X-ray image by using the detected X-rays.

The X-ray generator 120 receives power from the power supply 110 for generating X-rays and irradiates the generated X-rays toward a subject. The intensity and amount of X-ray irradiation depend on power and supply time supplied from the power supply 110.

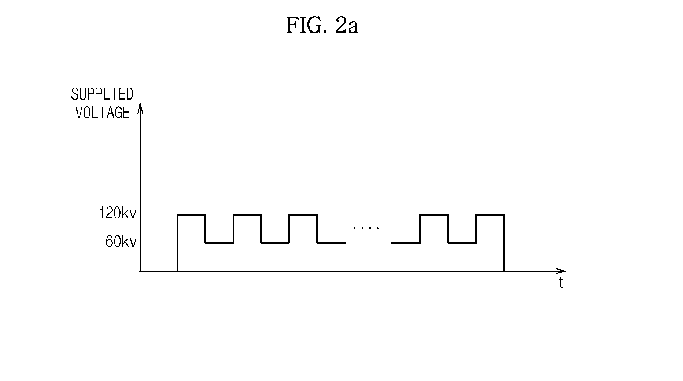

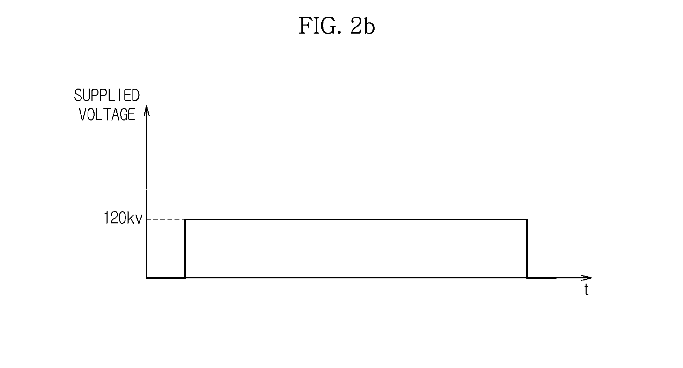

The power supply 110 supplies power to the X-ray generator 120, which power is used for determining an energy intensity of the X-rays, and a graph associated therewith is shown in FIG. 2A and FIG. 2B.

FIG. 2A is a graph which illustrates a level of power supplied from a power supply to an X-ray generator in a computed tomography apparatus of the related art. FIG. 2B is a graph which illustrates a level of power supplied from a power supply to an X-ray generator in a computed tomography apparatus according to an exemplary embodiment.

Referring to FIGS. 2A and 2B, the power supply 110 according to an exemplary embodiment continuously supplies a voltage having a predetermined constant level to the X-ray generator 120, while the power supply of the related art switches between high voltages and low voltages at a high speed for multi-energy tomography. Because X-rays generated at a high voltage have a characteristic spectrum which includes a low energy region and a high energy region, X-rays having a desired energy level can be transmitted to the subject via filtering.

The X-ray filter 130 is interposed between the X-ray generator 120 and the subject and includes at least one filter member which filters an X-ray having an energy level which falls within a predetermined energy range.

In particular, the X-ray filter 130 is disposed in an irradiation path of X-rays generated by the X-ray generator 120. After the X-rays generated by the X-ray generator 120 pass through the X-ray filter 130, an X-ray having an energy level which falls within a predetermined energy range is filtered and then irradiated and transmitted to the subject.

The at least one filter member of the X-ray filter 130 should be selectively disposed in the irradiation path of X-rays in order to switch between a high-energy X-ray mode and a low-energy X-ray mode. For this reason, the X-ray filter 130 rotates at a predetermined rotational speed. A detailed configuration of the X-ray filter 130 will be described below.

The driver 160 rotates the X-ray filter 130 and includes a motor. In an example of the driver 160 that rotates the X-ray filter 130, the X-ray filter 130 includes a rotation shaft, and the rotation shaft of the driver 160 is connected to a rotation shaft of the X-ray filter 130 in order to rotate the X-ray filter 130 via rotation of the motor.

The detector 140 detects X-rays which are irradiated toward the subject. The X-rays irradiated from the X-ray generator 120 traverse the body of the subject and the X-rays are attenuated while traversing the body of the subject. Because the transmittance of X-rays varies based on respective types of tissues constituting portions at which X-rays are irradiated, the amount of detectable X-rays varies depending on the position at which X-rays are irradiated.

The types of tissues that have different transmittance to X-rays can be roughly divided into soft tissues such as fat tissues, muscles, and blood, and calcium-rich tissues such as bones and teeth, and the body. Accordingly, depending on whether an X-ray is irradiated to a bone or a soft tissue of the subject, and the body or fat tissue thereof, the amount of detectable X-rays varies, and is the detected X-rays are converted into a signal which is used to obtain an X-ray image.

The detector 140 includes a common image intensifier and a charge-coupled device (CCD) camera. The detector 140 detects X-rays which are irradiated toward the subject, intensifies an image by using the image intensifier, converts the intensified image into an electric signal, and transmits the electric signal to the host apparatus 150.

The host apparatus 150 performs overall control of the computed tomography apparatus in order to obtain an X-ray image. In particular, the host apparatus 150 of the computed tomography apparatus, according to an exemplary embodiment, obtains X-ray images from X-rays detected by the detector 140, separates the obtained X-ray images based on a particular filter member from among the at least one filter member by which the corresponding X-rays are filtered, and reconstructs images based on a respective X-ray energy range by using X-ray images corresponding to the particular filter member. A detailed configuration and operation of the host apparatus 150 will be described in detail below.

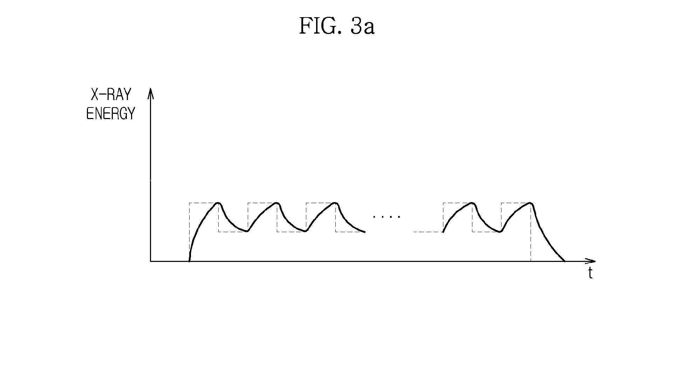

FIG. 3A is a graph which illustrates an energy profile of an X-ray irradiated from an X-ray generator of a computed tomography apparatus of the related art to a subject, and

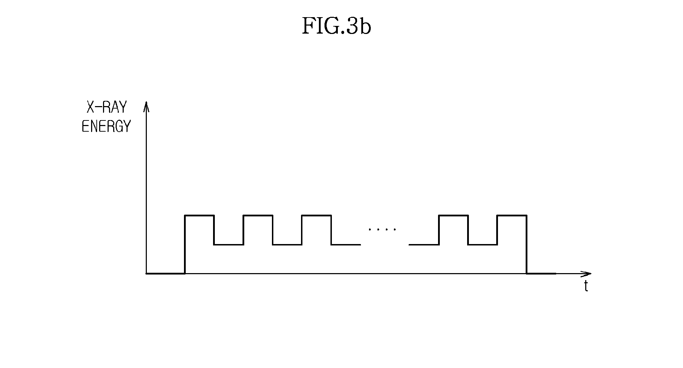

FIG. 3B is a graph which illustrates an energy profile of an X-ray passing through the X-ray filter 130 of the computed tomography apparatus according to an exemplary embodiment.

A graph showing a state in which an intensity of voltage is switching periodically is illustrated in FIG. 2A. Although a voltage is supplied as in FIG. 2A, the periodic switching of voltage from a high voltage to a low voltage, or from a low voltage to a high voltage, may cause difficulties. In particular, the X-ray energy irradiated from the X-ray generator 120 generally does not switch at the same rate as the switching of the supply voltage, and therefore, it takes a considerable time to switch an amount of X-ray energy from a high energy to a low energy, or from a low energy to a high energy, as shown in FIG. 3A. For this reason, a resulting X-ray image quality may be deteriorated. However, when the X-ray irradiated from the X-ray generator 120 passes through the X-ray filter 130 according to an exemplary embodiment, an X-ray energy level may be quickly switched between a high energy and a low energy, as shown in FIG. 3B. For this reason, a resulting X-ray image quality is improved.

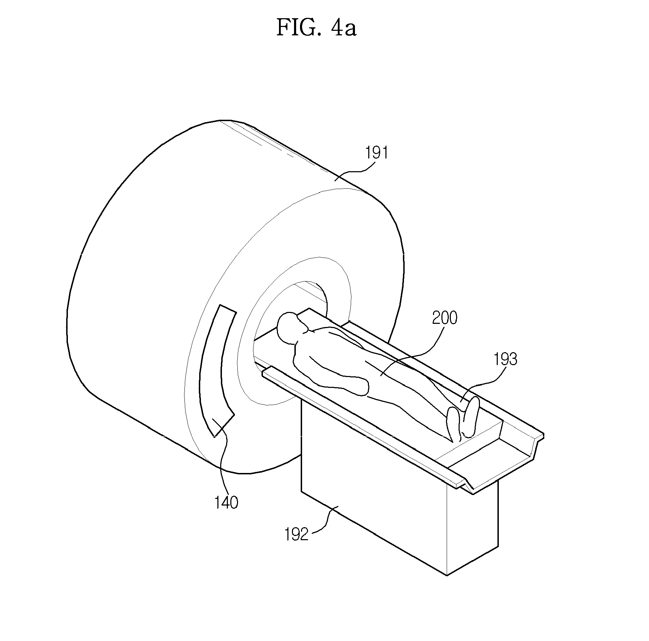

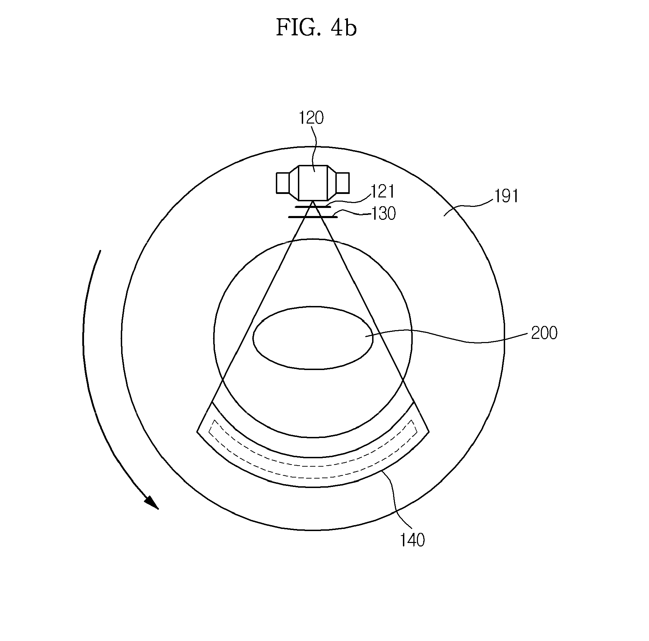

FIG. 4A is a perspective view which illustrates a computed tomography apparatus according to an exemplary embodiment. FIG. 4B is a cross-sectional view which illustrates a computed tomography apparatus according to an exemplary embodiment.

Referring to FIGS. 4A and 4B, the computed tomography apparatus according to an exemplary embodiment includes a gantry 191 including an X-ray generator 120 and a detector 140, and an inspection table 192 which is used to accept and transfer a subject 200.

The gantry 191 is provided in the center thereof with a cylindrical opening, and the subject 200 is inserted into the opening. The gantry 191 is provided with the X-ray generator 120 and the detector 140 such that the X-ray generator 120 faces the detector 140. Further, the X-ray generator 120 and the detector 140 are disposed in portions of the gantry 191 which enable detection of an X-ray that is vertically incident to the subject 200 that is inserted into the opening of the gantry 191.

When the subject 200 is inserted into the opening of the gantry 191 by using a transfer unit 193 on the inspection table 192, as the gantry 191 rotates by 360 degrees or by a predetermined angle with respect to the subject 200, it is possible to obtain cross-sectional images at various angles by using the X-ray generator 120 and the detector 140.

Further, the transfer unit 193 and the gantry 191 horizontally drive forwardly and backwardly, based on the same shaft, such that an inspection site of the subject 200 is disposed between the X-ray generator 120 and the detector 140. Still further, the gantry 191 rotates around the opening, based on the subject 200 as a central point, by using a gantry drive tool (not shown).

A collimator 121 is provided at the front surface of the X-ray generator 120 in the X-ray irradiation direction in order to enable X-rays to be irradiated toward the subject 200 in the form of a fan without being scattered in other directions. Further, although not shown, a collimator is also provided on the front surface of the detector 140 in order to enable X-rays to be detected only in a desired region.

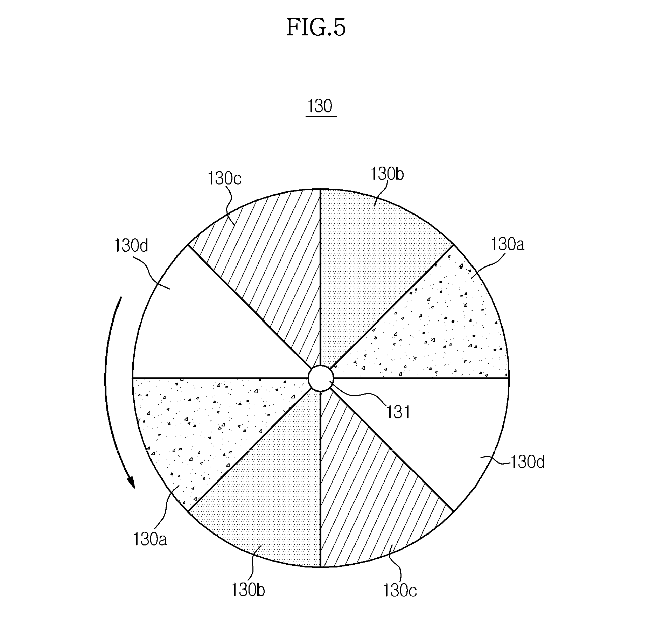

FIG. 5 is a front view which illustrates a configuration of an X-ray filter of the computed tomography apparatus according to an exemplary embodiment.

Referring to FIG. 5, the X-ray filter 130 of the computed tomography apparatus according to an exemplary embodiment may have a circular shape and may be provided in the center thereof with a rotation shaft 131. The driver 160 is connected to the rotation shaft 131 in order enable the driver 160 to rotate the X-ray filter 130.

The X-ray filter 130 may be divided into a plurality of regions, and each region may include a filter member which filters an X-ray having an energy level which falls within a predetermined energy range. The respective filter members may be different or identical. Alternatively, a particular region may have a hollow without including a filter member, thereby resulting in a region through which X-rays generated by the X-ray generator 120 pass without being filtered. Further, the X-ray filter 130 may include a blocking material which blocks X-rays.

In the embodiment shown in FIG. 5, the X-ray filter 130 is divided into eight regions and includes a first filter member 130a, a second filter member 130b, a third filter member 130c and a fourth filter member 130d.

As described above, the X-ray filter 130 is disposed between the X-ray generator 120 and the subject 200, and respective filter members 130a, 130b, 130c, and 130d are selectively arranged in an X-ray irradiation path, while the X-ray filter 130 rotates.

A material which is selected for use as the X-ray filter member may include copper, aluminum, or the like, and other materials may be selected depending on the intensity of X-rays to be filtered. Further, the intensity of X-rays to be filtered may be controlled by varying the thickness of the material. Copper and aluminum are provided only as an exemplary embodiment of the filter member used herein, and any material may be used without limitation so long as it is capable of filtering X-rays. Still further, the X-ray energy level to be filtered may also be arbitrarily selected by a user or designer, and is not limited to a specific energy level.

In one exemplary embodiment, the first filter member 130a filters out low-energy X-rays, and the second filter member 130b filters out low-energy X-rays having a respective energy level which is higher than the corresponding energy level of the X-rays filtered by the first filter member 130a. Further, the third filter member 130c is made of an X-ray blocker which blocks X-rays and the fourth filter member 130d is provided in the form of a hollow, thereby enabling X-rays generated by the X-ray generator 120 to pass therethrough. The filter member 130c which is made of an X-ray blocker may be also referred to as a "filter material", although it is substantially not used as a filter material in the sense of permitting a selected range of x-rays to pass therethrough.

The X-ray blocker which corresponds to the third filter member 130c blocks X-rays generated by the X-ray generator 120 and thereby reduces an amount of radiation to which the subject 200 is exposed while computed tomography is performed.

When X-rays generated by the X-ray generator 120 pass through the fourth filter member 130d, an X-ray having an energy level which falls within an energy spectrum which includes both of a low energy range and a high energy range is irradiated to the subject 200. The irradiated X-ray may have an energy level which is lower than a corresponding X-ray which passes through either of the first filter member 130a and the second filter member 130b.

Accordingly, as the X-ray filter 130 rotates at a high speed when an X-ray is generated and irradiated by the X-ray generator 120, the filter member disposed in the X-ray irradiation path is changed at a high speed, and an X-ray irradiated to the subject 200 is switched at a high speed in the following order: First, an X-ray having a first high energy based on first filter member 130a; second, an X-ray having a second high energy which is higher than the first high energy based on second filter member 130b; third, no X-rays based on X-ray blocking member 130c; and fourth, an X-ray having a low energy based on the hollow which corresponds to fourth filter member 130d. In this manner, without switching a voltage of the power supply 110 or providing the X-ray generator 120 and the X-ray detector 140 in plural, X-rays having different respective energy levels can be switched at a high speed.

Although a low-energy X-ray is obtained by passing X-rays through a hollow 130d of the X-ray filter 130 in the present exemplary embodiment, a low-energy X-ray may alternatively be obtained by providing a filter member which filters out an X-ray having a high energy level in the X-ray filter 130.

Further, specific items relating to an inner configuration of the X-ray filter 130, such as, for example, the number of regions constituting the X-ray filter 130 or the disposition order of filter members, are not limited, and these factors may be determined while taking into consideration the inspection site, inspection efficiency of the subject 200 and the like.

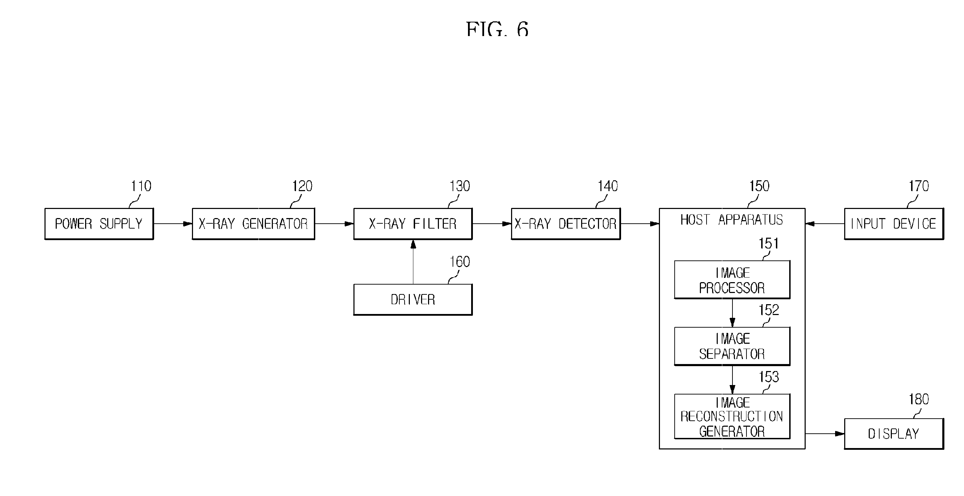

FIG. 6 is a block diagram which illustrates a controlled configuration of a computed tomography apparatus according to an exemplary embodiment.

The power supply 110, the X-ray generator 120, the X-ray filter 130, the X-ray detector 140 and the driver 160 are described with respect to FIG. 1 above. Accordingly, a detailed description thereof will be omitted.

The host apparatus 150 of the computed tomography apparatus according to an exemplary embodiment includes an image processor 151 which converts X-ray transmission information into digital image information, based on an electric signal received from the X-ray detector 140, an image separator 152 which separates X-ray images based on the filter members corresponding thereto, and an image reconstruction generator 153 which reconstructs tomographic images at respective filter members by using the separated images. Each of the image processor 151, the image separator 152, and the image reconstruction generator 153 may be implemented as a hardware component, such as, for example, an integrated circuit or dedicated circuitry; or as a software module which includes a software program which is executable by a computer or by a microprocessor; or as a combination of hardware and software.

The image processor 151 converts transmission information relating to X-rays converted into an electric signal into digital image information which may be used to obtain an X-ray image. In one exemplary embodiment, an amount of time which is required for obtaining X-ray images relates to a rotation time of the X-ray filter 130, and a detailed explanation thereof will be described below.

The computed tomography apparatus according to an exemplary embodiment further includes an input device 170 and a display 180. The input device 170 receives overall commands associated with control of the computed tomography apparatus from a user and transmits the received commands to the host apparatus 150. The display 180 displays an X-ray image which is obtained by the host apparatus 150 or a tomographic image which is reconstructed from the X-ray image to enable the user to diagnose a health state of the subject 200.

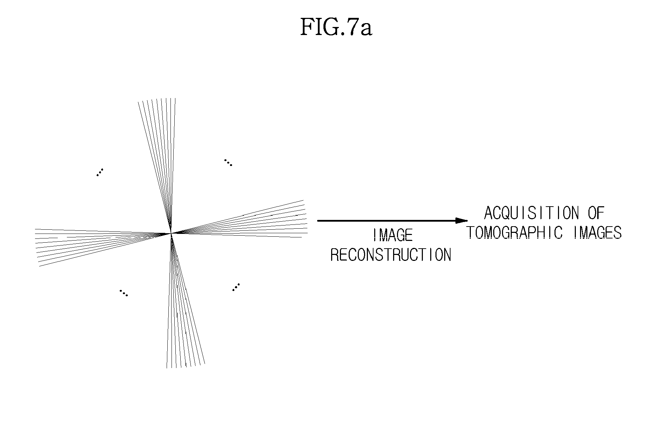

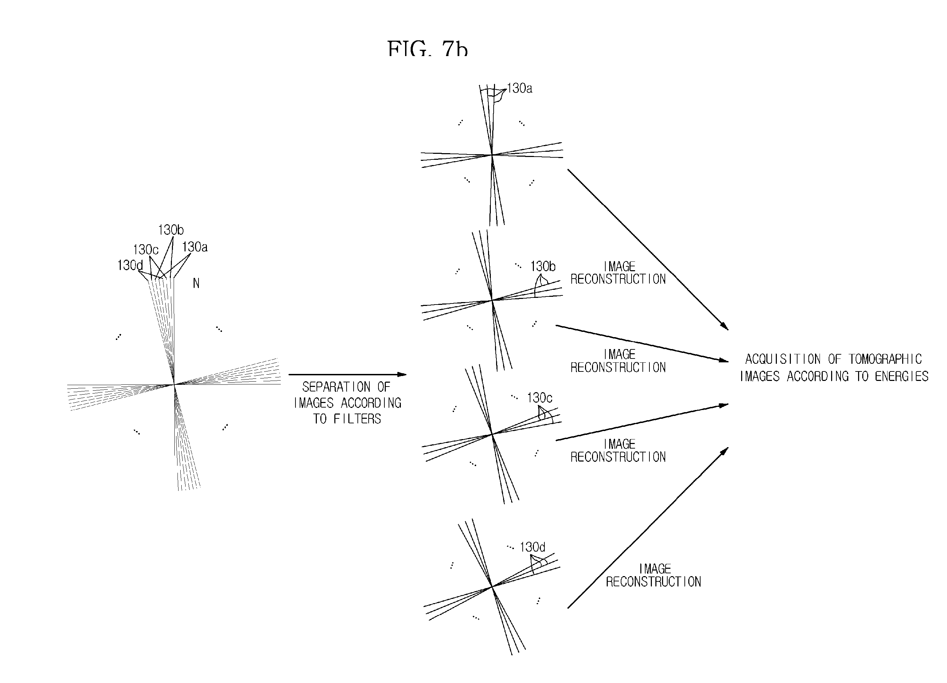

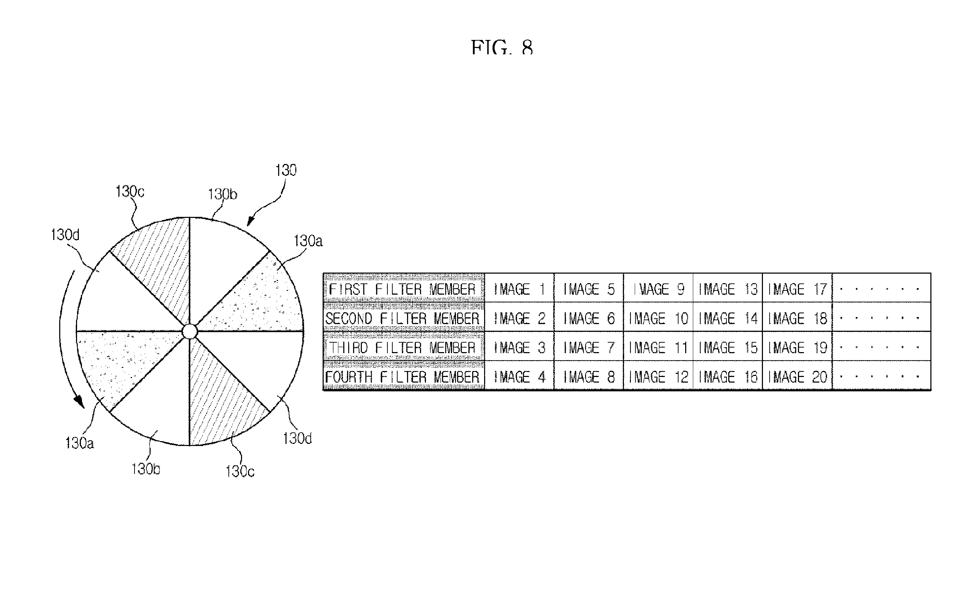

FIG. 7A and FIG. 7B are diagrams which illustrate images relating to X-rays that pass through respective filter members, which images are separated at the respective filter members. FIG. 8 is a sectional view of the X-ray filter 130 and an accompanying chart which illustrates respective images of X-rays of FIGs. 7A and 7B with respect to the corresponding filter members and the corresponding acquisition angles of the X-ray images.

Hereinafter, image separation and reconstruction of the computed tomography apparatus according to an exemplary embodiment will be described in detail with reference to FIGS. 7A, 7B, and 8.

In the present exemplary embodiment, the X-ray filter 130 shown in FIG. 5 is used.

The straight lines shown in FIGS. 7A and 7B represent X-rays that are irradiated from the X-ray generator 120 and pass through the X-ray filter 130.

Referring to FIG. 7A, in the computed tomography apparatus of the related art, the X-ray generator continuously irradiates X-rays while rotating around a subject by 360 degrees in order to continuously obtain X-ray images, and the computed tomography apparatus reconstructs tomographic images using all the obtained images.

Referring to FIG. 7B, the computed tomography apparatus according to an exemplary embodiment irradiates an X-ray that has passed through the first filter member 130a, an X-ray that has passed through the second filter member 130b, an X-ray that has passed through the third filter member 130c and an X-ray that has passed through the fourth filter member 130d in this order. The order of the irradiated X-rays will be described again in FIG. 8.

X-ray images for the corresponding X-rays are obtained in accordance with the order of irradiated X-rays. The image separator 152 separates the obtained X-ray images based on the respective filter members corresponding thereto. For example, when a total of 1000 X-ray images are obtained while the gantry 191 rotates once, from among the 1000 images, images obtained from X-rays that pass through the first filter member 130a are separated and, in the same manner, images obtained from X-rays that pass through the fourth filter member 130d are separated.

Referring to the X-ray filter 130 shown in FIG. 8, when the X-ray filter 130 rotates counterclockwise under the condition that the first filter member 130a is first disposed in the X-ray irradiation path, X-rays are filtered through each of the first filter member 130a, the second filter member 130b, the third filter member 130c and the fourth filter member 130d in this order. The rotation direction of the X-ray filter 130, and a disposition order, a respective type of each filter member, and a number of the filter members may be determined by a user or designer in accordance with a desired application.

Further, when the obtained images are separated and grouped according to respective filter members, X-ray images that belong to the group relating to the first filter member 130a include an image 1, an image 5, an image 9, ... and an image 997; X-ray images that belong to the group relating to the second filter member 130b include an image 2, an image 6, an image 10,..., and an image 998; X-ray images that belong to the group relating to the third filter member 130c include an image 3, an image 7, an image 11,..., and an image 999; and X-ray images that belong to the group relating to the fourth filter member 130d include an image 4, an image 8, an image 12,...., and an image 1000. The number assigned to each respective image indicates a corresponding sequential rank within the order in which the respective image was acquired. Accordingly, the image separator 152 separates X-ray images based on the filter members, and more particularly, based on a disposition order of the filter members and the acquisition order of the X-ray images.

Further, referring to FIG. 7B, when X-ray images are separated based on the respective filter members and tomographic images are reconstructed using the corresponding images, a classification of the tomographic images based on a respective energy level can be obtained. Specifically, when X-ray images that belong to the group relating to the first filter member 130a are reconstructed, tomographic images of an X-ray having an energy level which falls within a first high energy range can be obtained, and when X-ray images that belong to the group relating to the second filter member 130b are reconstructed, tomographic images of an X-ray having an energy level which falls within a second high energy range can be obtained, and when X-ray images that belong to the group relating to the fourth filter member 130d are reconstructed, tomographic images of an X-ray having an energy level which falls within a low energy range can be obtained.

Further, the host apparatus 150 can obtain tomographic images which exhibit superior contrast from tomographic images obtained from X-rays having different energy levels by using dual energy subtraction or the like, based on the fact that an attenuation property depends on X-ray intensity.

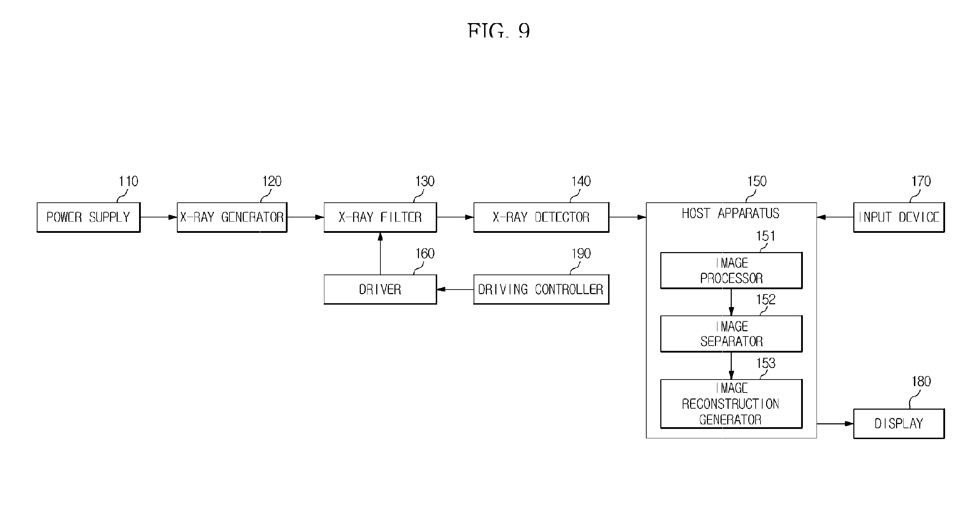

FIG. 9 is a block diagram which illustrates a controlled configuration of a computed tomography apparatus according to an exemplary embodiment.

Referring to FIG. 9, the computed tomography apparatus according to an exemplary embodiment may further include a driving controller 190 which controls the driver 160. Descriptions of the remaining components, that is, the power supply 110, the X-ray generator 120, the X-ray filter 130, the X-ray detector 140, the host apparatus 150, the driver 160, the input device 170, and a display 180 are provided with respect to FIG. 6 above and are thus omitted.

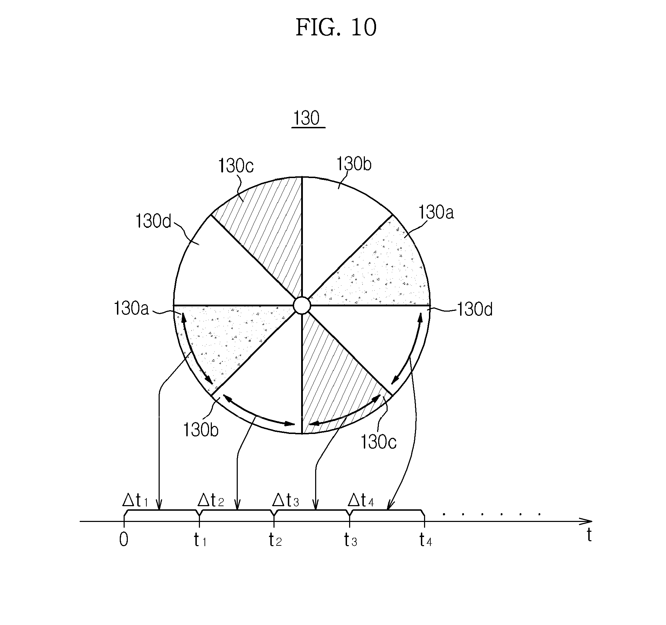

The driving controller 190 controls the driver 160 and ultimately controls a rotational speed of the X-ray filter 130. In particular, the driving controller 190 controls such that the rotational speed of the X-ray filter 130 relates to the time required for obtaining at least one X-ray image, and will be described in detail with reference to FIG. 10.

FIG. 10 is a view which illustrates a relation between a rotational speed of the X-ray filter 130 and an image acquisition time.

The X-ray filter 130 of the exemplary embodiment shown in FIG. 10 is the same as that shown in FIG. 5, and a time axis illustrated at the lower part of FIG. 10 represents a time required for obtaining an X-ray image.

When the first filter member 130a that belongs to a lower semicircle of the X-ray filter 130 is first disposed in an irradiation path of X-rays and rotates clockwise, time Δt1 represents a time required for obtaining an image from an X-ray that passes through the first filter member 130a, and Δt2 represents a time required for obtaining an image from an X-ray that passes through the second filter member 130b. In this exemplary embodiment, the time required for obtaining an X-ray image is the same, regardless of X-ray intensity (i.e., Δt1=Δt2=···=Δt).

When the filter member changes during acquisition of an X-ray image, the energy of the corresponding detected X-ray also changes, and interference may thus occur. Accordingly, the driving controller 190 of the computed tomography apparatus according to an exemplary embodiment controls a rotational speed of the X-ray filter 130 such that the filter member does not change while one X-ray image is being obtained.

In particular, a time interval during which an X-ray generated by the X-ray generator 120 passes through a point A of the first filter member 130a and then reaches a point B is synchronized with Δt1. Further, a time interval during which an X-ray generated by the X-ray generator 120 passes through a point A of the second filter member 130b and then reaches a point B is synchronized with Δt2. The same synchronization is also applied to each of the third filter member 130c and the fourth filter member 130d. When Δt1=Δt2=···=Δt is applied in conjunction with the condition that a respective size of each of the respective filter members is identical, the driving controller 190 controls a rotational speed of the X-ray filter 130 such that a time interval during which an X-ray irradiated from the X-ray generator 120 passes through one filter member is the same as a time Δt required for obtaining an X-ray image. Such speed control may be autonomously carried out in the driving controller 190, or by mutual reception of data between the driving controller 190 and a host apparatus 150 (not shown) connected thereto.

Hereinafter, exemplary embodiments of a method for controlling the computed tomography apparatus according to an exemplary embodiment will be described.

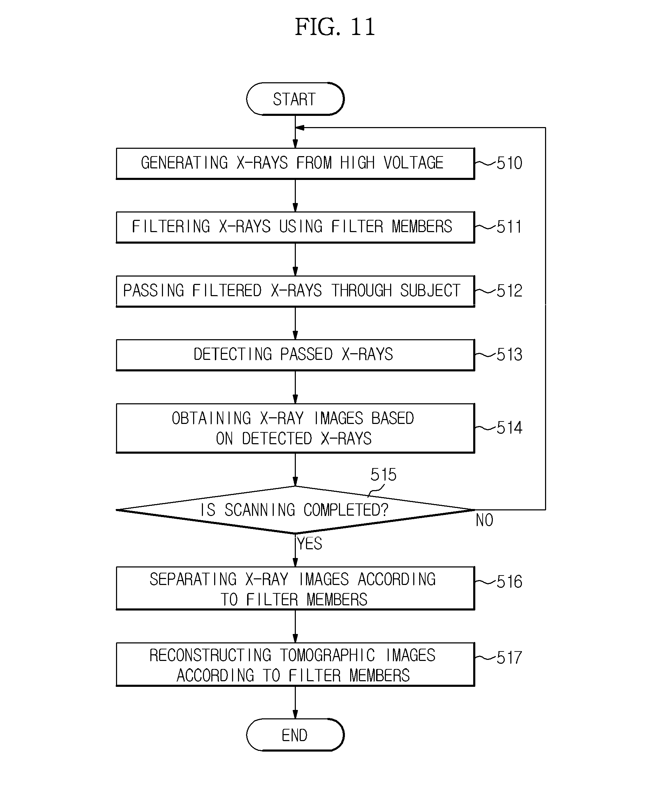

FIG. 11 is a flowchart which illustrates a method for controlling the computed tomography apparatus according to an exemplary embodiment.

Referring to FIG. 11, first, in operation 510, a voltage having a predetermined magnitude is supplied to the X-ray generator 120, thereby causing the X-ray generator 120 to generate an X-ray. The X-ray filter 130 is disposed between the X-ray generator 120 and the subject 200 and, from among filter members constituting the X-ray filter 130, a filter member disposed in an X-ray irradiation path filters an X-ray irradiated from the X-ray generator 120 in operation 511.

In operation 512, the filtered X-ray passes through the subject, and then, in operation 513, the X-ray detector 140 detects the transmitted X-ray. In operation 514, the host apparatus 150 obtains an X-ray image based on transmission information relating to the X-rays detected by the detector 140. The detector 140 sends X-ray transmission information in the form of an electric signal to the host apparatus 150, and the host apparatus 150 converts the electric signal into digital image information in order to obtain an X-ray image.

In operation 515, a determination is made as to whether or not scanning has been completed, and in particular, whether or not a rotation of the gantry 191 has been completed is determined. When scanning is not completed (i.e., a determination of NO is made in operation 515), an X-ray is generated and a process for obtaining an X-ray image is repeated. In an exemplary embodiment, generation of an X-ray by using a power supply which supplies a high voltage is continuously performed. When a completion of scanning is determined (i.e., a determination of YES is made in operation 515), a supply of power to the X-ray generator 120 is stopped, and when scanning is determined to be not completed (i.e., a determination of NO is made in operation 515), supply of a high voltage is maintained.

Further, during filtering of the X-ray in operation 511, the filter members are changed in order of a first filter member, second filter member, ...., in accordance with a corresponding rotation of the X-ray filter 130. In an exemplary embodiment, the rotational speed of the X-ray filter 130 may relate to an amount of time required for obtaining an X-ray image. A detailed explanation thereof has been provided above with reference to FIG. 10.

When a process of generating an X-ray and obtaining an X-ray image is repeated and scanning is completed (i.e., a determination of YES is made in operation 515), the image separator 152 separates X-ray images based on respective filter members in operation 516. Accordingly, in operation 516, X-ray images are separated based on respective filter members through which corresponding X-rays, the basis of X-ray images, pass. This process uses order of the filter members disposed in the X-ray filter 130 and a corresponding acquisition order of X-ray images. A detailed explanation thereof has been provided above with reference to FIGS. 7A, 7B, and 8.

For reference, in another exemplary embodiment, X-ray images are stored in assigned memories in order to separate the X-ray images after X-ray images are obtained. In particular, the X-ray images may be separated based on the respective filter members, without an additional image separation process, after completion of scanning, such that an X-ray image obtained from an X-ray that has passed through the first filter member 130a is stored in a first memory, an X-ray image obtained from an X-ray that has passed through the second filter member 130b is stored in a second memory, an X-ray image obtained from an X-ray that has passed through the third filter member 130c is stored in a third memory, and an X-ray image obtained from an X-ray that has passed through the fourth filter member 130d is stored in a fourth memory.

In operation 517, each respective one of the filter members independently reconstructs tomographic images. Depending on the respective filter member through which a particular X-ray is filtered, the energy intensity of the particular X-ray varies correspondingly. Accordingly, reconstruction of tomographic images based on the respective filter members is the same as reconstruction of tomographic images based on corresponding X-ray energy levels. Because X-ray images have been separated based on the filter members, tomographic images may be reconstructed based on the respective filter members by using the separated X-ray images.

According to exemplary embodiments as described above, it is possible to switch X-rays having different energy levels without switching a voltage level of a power supply and without providing a plurality of X-ray generators, and it is possible to separate X-ray images based on respective X-ray energy levels based on filter member type and thus to efficiently obtain tomographic images based on different X-ray energy levels using the same. Further, it is possible to prevent interference caused by variations in energy intensity, because an acquisition time of X-ray images relates to the rotational speed of X-ray filters.

In a computed tomography apparatus and a method for controlling the same according to exemplary embodiments, it is possible to switch between a high energy mode and a low energy mode at a high speed by using one X-ray generator and an additional filter that rotates at a high speed, and to improve efficiency with respect to both cost and time due to a reduced need for the additional adjustment process.

Further, it is possible to prevent generation of noise between detectors because a plurality of X-ray images are not obtained simultaneously, and it is possible to obtain uniform results at respective energy ranges because voltage of an X-ray generator is not switched.

Further, it is possible to prevent interference which would otherwise be caused by variations in energy intensity, because acquisition time of X-ray images is linked to the rotation speed of X-ray filters.

In an exemplary embodiment, a program and/or a code for performing the above-described methods may be stored on various types of terminal-readable recording media such as a random access memory (RAM), a flash memory, a read only memory (ROM), an erasable programmable ROM (EPROM), an electronically erasable and programmable ROM (EEPROM), a register, a hard disk, a removable disk, a memory card, a USB memory, a CD-ROM, an/or any other suitable non-transitory or transitory medium.

Although a few exemplary embodiments have been shown and described, it will be appreciated by those skilled in the art that changes may be made in these exemplary embodiments without departing from the principles and spirit of the present disclosure, the scope of which is defined in the claims and their equivalents.

## Claims

1. A computed tomography apparatus comprising:
an X-ray generator which generates X-rays and irradiates the generated X-rays toward a subject;
an X-ray filter disposed between the X-ray generator and the subject, the X-ray filter including at least one filter member which filters an X-ray having an energy level which falls within a predetermined energy range;
a driver which rotates the X-ray filter such that the at least one filter member is selectively disposed in an irradiation path of X-rays generated by the X-ray generator;
a detector which detects the X-rays that are irradiated by the X-ray generator and filtered by the X-ray filter; and
a host apparatus which obtains X-ray images by using the X-rays detected by the detector, separates the obtained X-ray images based on a particular filter member from among the at least one filter member by which the corresponding X-rays are filtered, and reconstructs the separated images based on a respective X-ray energy range by using X-ray images corresponding to the particular filter member.

2. The computed tomography apparatus according to claim 1, wherein the host apparatus separates the obtained X-ray images based on a disposition order of the at least one filter member and an acquisition order of the X-ray images.

3. The computed tomography apparatus according to claim 1, wherein a rotational speed of the X-ray filter corresponds to an amount of time required for obtaining at least one X-ray image by the host apparatus.

4. The computed tomography apparatus according to claim 3, wherein a time at which the at least one X-ray filter member is disposed in the X-ray irradiation path is synchronized with the amount of time required for obtaining the at least one X-ray image by the host apparatus.

5. The computed tomography apparatus according to claim 1, wherein the X-ray filter has a circular shape and includes a rotation shaft which is rotated by the driver, and wherein the at least one X-ray filter member is disposed based on the rotation shaft.

6. The computed tomography apparatus according to claim 1, wherein the X-ray generator receives a voltage having a predetermined level for generating an X-ray.

7. The computed tomography apparatus according to claim 6, wherein the X-ray filter includes an X-ray blocker which blocks X-rays.

8. A method for controlling a computed tomography apparatus, the method comprising:
generating X-rays and irradiating the generated X-rays toward a subject;
filtering the irradiated X-rays by using an X-ray filter that includes at least one filter member which filters an X-ray having an energy level which falls within a predetermined energy range and which rotates at a predetermined rotational speed;
detecting the filtered X-rays;
obtaining X-ray images by using the detected X-rays;
separating the obtained X-ray images based on a particular filter member from among the at least one filter member which filters the corresponding X-rays; and
reconstructing the separated images based on a respective X-ray energy range by using X-ray images corresponding to the particular filter member.

9. The method according to claim 8, wherein the obtained X-ray images are separated based on a disposition order of the at least one filter member and an acquisition order of the X-ray images.

10. The method according to claim 8, wherein the predetermined rotational speed of the X-ray filter corresponds to an amount of time required for obtaining at least one X-ray image.

11. The method according to claim 10, wherein a time at which the at least one X-ray filter member is disposed in an X-ray irradiation path is synchronized with the amount of time required for obtaining the one X-ray image.

12. The method according to claim 8, wherein the X-ray filter has a circular shape and includes a rotation shaft which is rotated by a driver, and wherein the at least one X-ray filter member is disposed based on the rotation shaft.

13. A control device for use in conjunction with a computed tomography apparatus which includes a rotating filter and a detector, the control device comprising:
an image processor which obtains X-ray images by using X-rays which have been filtered by the filter, detected by the detector, and received from the detector;
an image separator which separates the obtained X-ray images into respective groups based on a respective filter member from among a plurality of filter members by which the X-rays used for each corresponding obtained X-ray image are filtered; and
an image reconstruction generator which reconstructs the separated images based on a respective X-ray energy range by using the X-ray images included in each respective group.

14. The control device according to claim 13, wherein the image separator separates the obtained X-ray images based on a disposition order of the plurality of filter members and an acquisition order of the X-ray images.

15. The control device according to claim 13, wherein an amount of time required for obtaining at least one X-ray image by the image processor is used to determine a rotational speed of filter.
